(19) Europäisches Patentamt
European Patent Office
Office européen des brevets

(11) **EP 3 705 158 B1**

(12) **EUROPÄISCHE PATENTSCHRIFT**

(45) Veröffentlichungstag und Bekanntmachung des Hinweises auf die Patenterteilung:
**07.08.2024  Patentblatt 2024/32**

(21) Anmeldenummer: **19168592.4**

(22) Anmeldetag: **11.04.2019**

(51) Internationale Patentklassifikation (IPC):
**A61N 1/375** (2006.01)

(52) Gemeinsame Patentklassifikation (CPC):
**A61N 1/375**

(54) **GEHÄUSE FÜR EIN IMPLANTIERBARES MEDIZINISCHES GERÄT**

HOUSING FOR AN IMPLANTABLE MEDICAL DEVICE

BOÎTIER POUR UN APPAREIL MÉDICAL POUVANT ÊTRE IMPLANTÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.03.2019  DE 102019105798**

(43) Veröffentlichungstag der Anmeldung:
**09.09.2020  Patentblatt 2020/37**

(73) Patentinhaber: **BIOTRONIK SE & Co. KG
12359 Berlin (DE)**

(72) Erfinder: **Dörr, Thomas
12437 Berlin (DE)**

(74) Vertreter: **Biotronik Corporate Services SE
Corporate Intellectual Property
Sieversufer 7-9
12359 Berlin (DE)**

(56) Entgegenhaltungen:
WO-A2-93/02742        US-A1- 2007 060 980
US-A1- 2016 263 384    US-B2- 9 123 470

**Beschreibung**

Die vorliegende Erfindung betrifft Gehäuse für implantierbare medizinische Geräte (Implantable Medical Device, IMD).

Gehäuse für IMDs beherbergen die wichtigsten Gerätekomponenten und schützen diese vor dem Umgebungsmilieu des Implantationsortes. Die Gerätegehäuse können Schnittstellen nach außen aufweisen, z.B. zur elektrischen und mechanischen Ankopplung von Gerätekomponenten (beispielsweise für Stimulationselektroden für Schrittmacherimplantate) oder Sensorschnittstellen zur Messung von Körperparametern.

Es gibt IMDs, deren Gerätegehäuse in einer Haut- oder Gewebetasche des Patienten implantiert werden (Zum Beispiel bei Herzschrittmacherimplantaten oder Rückenmarksstimulatoren). Die Gerätegehäuse sollten so klein wie möglich dimensioniert und so geformt sein, dass sie nicht vom Patienten als störend empfunden werden. Im Stand der Technik sind flache, abgerundete Gehäuseformen bekannt, die eine kleine Wölbung in der Haut verursachen, die möglichst wenig heraussteht. Die US2007/0060980A1 offenbart ein Gehäuse für einen Herzschrittmacher mit einem kleinen Querschnitt, der für eine minimal invasive Implantation geeignet ist.

Herzschrittmacher, implantierbare Kardioverter-Defibrillatoren (Implantable Cardioverter-Defibrillator, ICD) oder CRT (Cardiac Resynchronization Therapy) Geräte weisen in der Regel ein flaches, abgerundetes Gehäuse aus Metall auf. Das Gehäuse ist an der Stirnseite mit einer Header-Komponente (Header im Folgenden) mechanisch verbunden, die typischerweise aus gegossenem Epoxidharz besteht und die Anschlüsse für die Elektrodenleitungen bereitstellt. Elektrische Leitungen führen aus dem Gerätegehäuse in den Header zu den Anschlüssen für die Elektrodenleitungen und gewährleisten so die elektrische Verbindung zwischen Elektroden und den im Inneren des Gehäuses angeordneten Komponenten wie dem Geräteprozessor, der Batterie oder bei ICDs dem Schock-Kondensator. Das Gerätegehäuse mit Header wird im oberen Brustbereich, über den Rippen, unterhalb des Schlüsselbeins in einer Haut- oder Gewebetasche implantiert. Das Gerätegehäuse im Verbund mit dem Header wird im Folgenden als Gehäuseanordnung bezeichnet.

Im Stand der Technik sind Herzschrittmacher, ICDs und CRT-Geräte bekannt, bei denen die Gehäuseanordnung aus Gerätegehäuse und Header flach ausgebildet ist und eine kreisrunde Außenkontur aufweist. Ein Problem solcher Gehäuseanordnungen besteht darin, dass sie sich in der Haut- bzw. Gewebetasche leicht drehen kann. Die an den Header angeschlossenen Elektrodenleitungen werden dabei mitgedreht, wodurch ein Elektrodenbruch entstehen kann.

Auch bekannt sind Gehäuseanordnungen, bei denen das Gerätegehäuse breiter ist als hoch, oder höher ist als breit. Ein Nachteil solcher Gehäuseanordnungen besteht darin, dass das Gerätegehäuse dazu neigt, auf den Rippen des Patienten zu ‚kippeln'. Das Kippeln ist bedingt durch den zwischen den Rippen des Patienten und dem Gerätegehäuse existierenden Raum, der verhältnismäßig groß ist für solche Gehäuseanordnungen, da das Gerätegehäuse über eine relativ langgestreckte flache Seite verfügt, die nicht der Rippenkontur folgt. Insbesondere bei Patienten mit kleinem Brustkorb, deren Rippenkontur einen kleineren Kreisradius aufweisen, ist das ‚Kippeln' ausgeprägt und unangenehm.

Bei der Implantation solcher Gehäuseanordnungen wird ein vergleichsmäßig kleiner Schnitt in die Haut des Patienten gemacht, was den Nachteil mit sich bringt, dass der Arzt eingeschränkten Gestaltungsspielraum bei der Formung der Haut- bzw. Gewebetasche zur Verfügung hat.

Der Erfindung liegt die Aufgabe zugrunde, ein Gerätegehäuse für ein IMD bereitzustellen, das sich für den Patienten angenehm unter der Haut zu tragen ist, d.h. schmerzfrei und im Wesentlichen ohne spürbare Spann- und Zugkräfte. Auch ist eine Aufgabe der Erfindung, ein Gerätegehäuse für ein IMD bereitzustellen, das möglichst wenig ‚kippelt', wenn es über den Rippen des Patienten implantiert wird.

Eine weitere Aufgabe der Erfindung liegt darin, ein Gerätegehäuse für ein IMD bereitzustellen, das sich im implantierten Zustand nicht einfach verdreht.

Es ist eine weitere Aufgabe der Erfindung, ein Gerätegehäuse für ein IMD bereitzustellen, das sich einfach, schnell und kontrolliert implantieren lässt.

Die Aufgaben werden durch ein erfindungsgemäßes Gerätegehäuse nach Anspruch 1 sowie eine Gehäuseanordnung umfassend das Gerätegehäuse nach Anspruch 11 gelöst. Vorteilhafte Ausführungsbeispiele der Erfindung finden sich in den abhängigen Ansprüchen.

Gemäß der erfinderischen Lösung wird ein Gerätegehäuse für ein IMD vorgeschlagen, wie es in Anspruch 1 beschrieben ist.

Das vorschlagsgemäße Gerätegehäuse weist auf diese Weise eine optimale Geometrie auf, um dem Patienten ein angenehmes Tragegefühl zu ermöglichen, d.h. das ‚Kippeln' auf den Rippen wird vermieden bis stark gemindert, da das Gerätegehäuse über keine langgestreckte Fläche verfügt. Gleichzeitig wird ein Drehen in der Haut- bzw. Gewebetasche verhindert, da das Gehäuse nicht kreisrund ist.

Des Weiteren, gemäß vorteilhaften Aspekten der Erfindung, hat die Vorderseite eine maximale Breite (Bmax) von 60 mm.

Gemäß weiteren Ausführungsbeispielen der Erfindung hat die Vorderseite eine maximale Höhe (Hmax) einen Wert von 48 mm.

Die genannten Maße für Verhältnis R, Bmax oder Hmax haben sich als besonders effektiv für die Lösung der genannten Aufgaben gezeigt.

Des Weiteren, nach einem weiteren Aspekt der vorliegenden Erfindung, hat das Gerätegehäuse eine maximale Dicke Dmax, wobei die Dicke senkrecht zur Ebene der Vorderseite gemessen wird, und die maximale Dicke einen Wert von 9 mm bis 15 mm; oder 9 mm bis 12 mm, oder 10 mm bis 11 mm; oder von 10 mm aufweist.

Die genannten Dicken ermöglichen ein ergonomisches, dünnes Gerätegehäuse, bei ausreichend Innenvolumen für die Unterbringung der Gerätekomponenten, wenn andere vorschlagsgemäße Geometrien für das Gerätegehäuse in Kombination gewählt werden.

Nach weiteren vorteilhaften Ausführungsformen der erfinderischen Lösung weist das Gerätegehäuse eine Rückseite auf, die deckungsgleich und gegenüberliegend zur Vorderseite angeordnet ist, und wobei die ebene Stirnseite an eine gerade Oberkante der Rückseite anschließt.

Auch kann das Gerätegehäuse zwei Gehäuseschalen umfassen, wobei die erste Gehäuseschale die Vorderseite aufweist und die zweite Gehäuseschale die Rückseite aufweist, wobei die erste und zweite Gehäuseschale symmetrisch ausgebildet sind.

Auf diese Weise wird ein symmetrischer Aufbau des Gerätegehäuses ermöglicht. Ein symmetrischer Aufbau ist vorteilhaft für Patienten, die ein IMD rechtsseitig statt linksseitig implantiert bekommen. So wird das IMD mit dem erfindungsgemäßen Gerätegehäuse einfach umgedreht und kann spiegelseitig implantiert werden, dabei weist es für den Patienten dieselben Eigenschaften bezüglich des Tragekomforts auf, wie für einen regulären Patienten mit linksseitig implantiertem IMD.

Des Weiteren, nach vorteilhaften Aspekten der vorliegenden Erfindung, weist die Außenkontur der Vorderseite des Gerätegehäuses Kurven mit mindestens zwei unterschiedlichen Kurvenradien auf. Dabei wird ein maximaler Kurvenradius von 12 cm, oder 11 cm, oder 10 cm, oder 9,5 cm nicht überschritten.

Durch die Wahl der Kurvenradien wird sichergestellt, dass das Implantat über keine langgestreckte Fläche verfügt, sodass 'Kippeln' auf den Rippen verhindert wird.

Des Weiteren, kann das Verhältnis der Fläche der Vorderseite zu der Fläche einer Ellipse, die durch maximale Breite (Bmax) und maximale Höhe (Hmax) der Vorderseite aufgespannt wird, d.h.

$$\frac{\text{Fläche (Vorderseite)}}{\text{Fläche (Ellipse)}}$$

, höchstens 1,5; oder höchstens 1,4; oder höchstens 1,3; oder höchstens 1,2; oder höchstens 1,15; oder höchstens 1,1 beträgt.

Durch die Wahl der vorschlagsgemäßen 'Füllfaktoren' wird sichergestellt, dass zum einen die Gestalt des Gerätegehäuses 'kreisähnlich' oder zumindest 'ellipsenähnlich' bleibt, sodass spitze Ecken der Kontur und langgestreckte Flächen zu verhindern, um den Tragekomfort zu erhöhen und 'Kippeln' zu vermeiden.

Gemäß weiteren Aspekten der vorliegenden Erfindung weist das Gerätegehäuse abgerundete Kanten auf. Diese erhöhen den Tragekomfort für den Patienten.

Gemäß weiteren Ausführungsformen wird außerdem eine Gehäuseanordnung für ein IMD vorgeschlagen, das ein erfindungsgemäßes Gerätegehäuse sowie einen Header (3) umfasst. Der Header ist an der ebenen Stirnseite des Gerätegehäuses mit dem Gerätegehäuse verbunden.

Außerdem wird ein Verfahren, das nicht Teil der Erfindung ist, zur Implantation einer erfindungsgemäßen Gehäuseanordnung vorgeschlagen. Das Verfahren umfasst die Schritte:

- Bereitstellen der Gehäuseanordnung,
- Eröffnen des Körpergewebes eines Patienten unterhalb des Schlüsselbeins mittels eines Schnitts,
- Präparieren einer Gewebetasche von einer Größe, die in etwa dem Volumen der Gehäuseanordnung entspricht,
- Einbringen des Gerätegehäuses in die Gewebetasche,
- Verschließen der Gewebetasche.

Gemäß einem vorteilhaften Aspekt des offenbarten Verfahrens hat der Schnitt eine Länge kleiner gleich der maximalen Breite (Bmax) der Vorderseite des Gerätegehäuses. Alternativ kann der Schnitt eine Länge aufweisen, die kleiner gleich der maximalen Höhe (Hmax) der Vorderseite plus einer Höhe des Headers entspricht. Auf diese Weise wird ein Schnitt von optimaler Länge zur Präparation einer Haut- oder Gewebetasche appliziert. Weitere Vorteilhafte Ausführungsformen sind in den Figuren und Figurenbeschreibungen zu entnehmen. Die Figuren dienen zur Veranschaulichung unterschiedlicher Aspekte der Erfindung und sollen nicht als einschränkend für den Schutzgegenstand gesehen werden.

**Kurzbeschreibung der Figuren**

Figur 1    Schematische Ansicht einer Gehäuseanordnung gemäß der vorliegenden Erfindung

Figur 2    Schematische Seitenansicht einer Gehäuseanordnung gemäß der vorliegenden Erfindung

Figur 3    Schematische Darstellung für die Bestimmung des Kurvenradius der Kontur des Gerätegehäuses

Figur 4    Schematische Darstellung der Bestimmung eines Füllfaktors für die Fläche der Vorderseite des Gerätegehäuses

Figur 5    Schematische Darstellung der Bestimmung eines Füllfaktors für die Fläche der Vorderseite des Gerätegehäuses

**Detaillierte Beschreibung der Figuren**

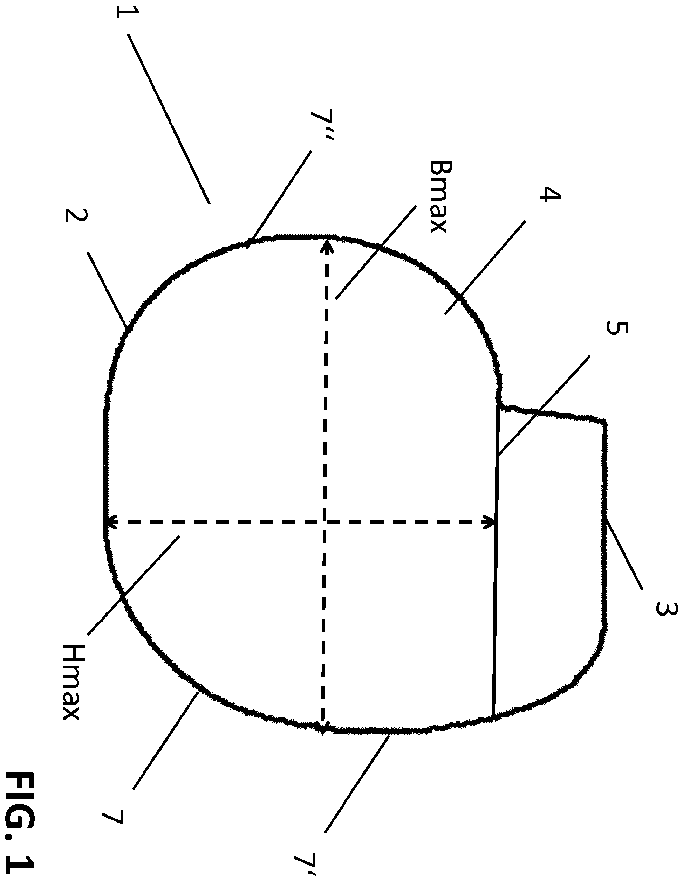

Figur 1 zeigt die Konturen einer erfindungsgemäßen Gehäuseanordnung 1, umfassend Gerätegehäuse 2 und Header 3. Gerätegehäuse 2 weist eine Vorderseite 4 sowie eine ebene Stirnseite 6, die senkrecht zu Vorderseite 4 angeordnet ist und an eine gerade Oberkante 5 der Vorderseite anschließt, auf. Die Vorderseite 4 weist eine maximale Breite Bmax auf, die parallel zur geraden Oberkante 5 gemessen wird. Vorderseite 4 weist außerdem eine maximale Höhe Hmax auf, die senkrecht zur geraden Oberkante 5 gemessen wird. Die Außenkontur der Vorderseite 4 umfasst Kurven mit mindestens zwei unterschiedlichen Kurvenradien 7, 7', 7''.

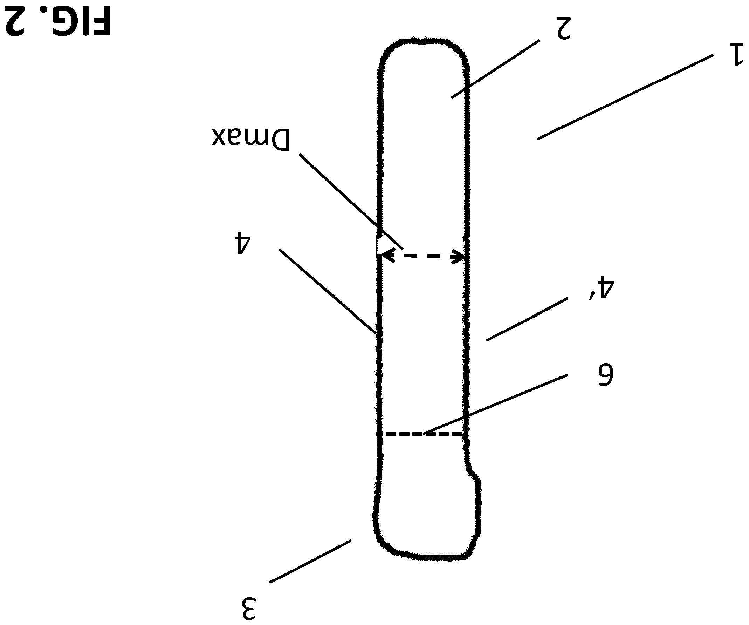

Figur 2 zeigt eine schematische Seitenansicht der erfindungsgemäßen Gehäuseanordnung. Zu sehen ist die maximale Dicke des Gerätegehäuses Dmax, die senkrecht zur Vorderseite 4 gemessen wird. Das Gerätegehäuse 2 weist außerdem eine Rückseite 4' auf, die deckungsgleich zur Vorderseite 4 gestaltet sein kann.

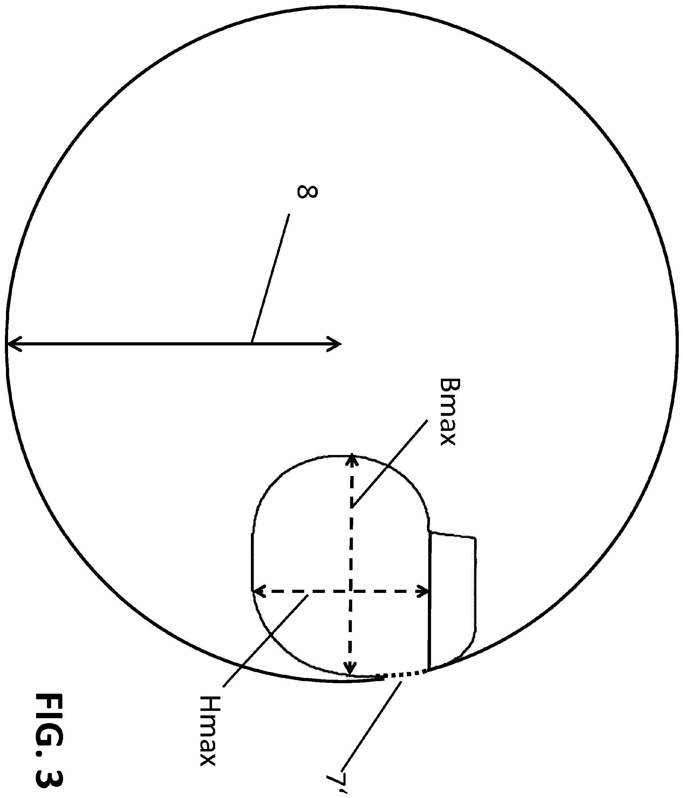

Figur 3 zeigt schematisch eine Messung des Kurvenradius 7'. Der korrespondierende Kreissegment ist gestrichelt gezeichnet. Ein Kreis mit entsprechendem Kreissegment wird überlagert und der Kreisradius 81 entspricht dem Kurvenradius 7'.

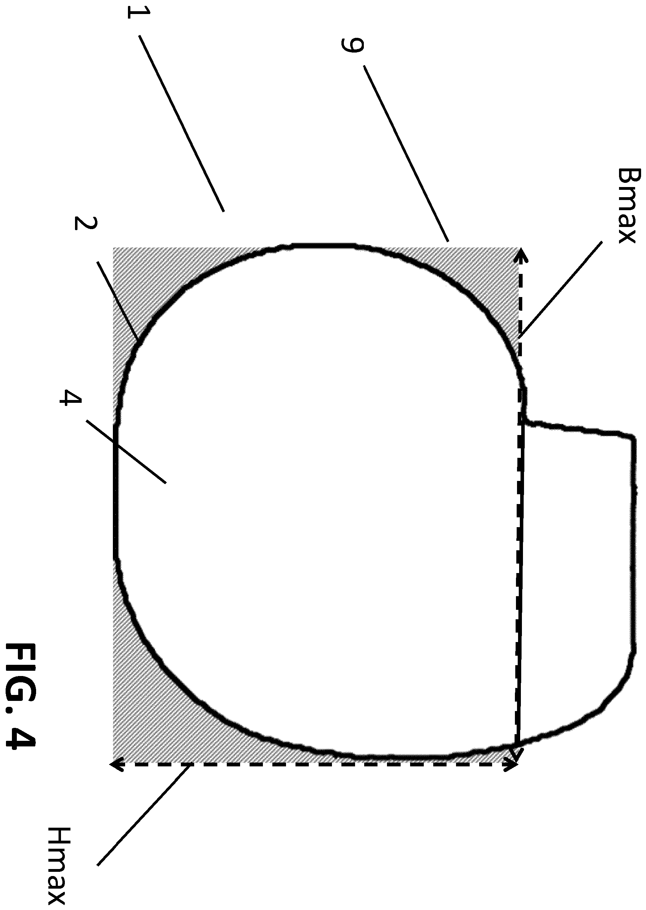

Figur 4 zeigt schematisch die Bestimmung des Füllfaktors, der gebildet wird durch das Verhältnis der Fläche von Vorderseite 4 zu einem Rechteck 9, das durch Bmax und Hmax aufgespannt wird. Die schraffierte Fläche entspricht dem Teil der Rechteckfläche, die nicht durch die Fläche der Vorderseite 4 ausgefüllt wird.

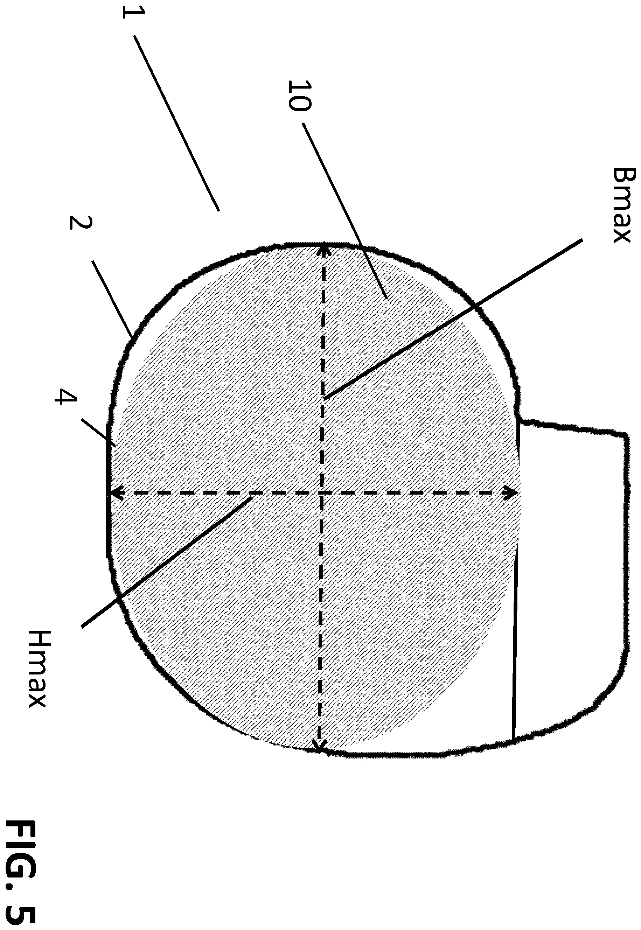

Figur 5 zeigt schematisch die Bestimmung des Füllfaktors, der gebildet wird durch das Verhältnis der Fläche von Vorderseite 4 zu einer Ellipse 10, die Bmax und Hmax als Hauptund Nebenachse aufweist. Die nicht durch die schraffierte Fläche abgedeckte Fläche der Vorderseite 4 entspricht dem Teil der Fläche, die über jene der Ellipse hinausragt.

**Patentansprüche**

1. Gerätegehäuse (2) für ein IMD, aufweisend:

   - eine Vorderseite (4),
   - eine ebene Stirnseite (6), die senkrecht zu Vorderseite (4) angeordnet ist und an eine gerade Oberkante (5) der Vorderseite anschließt,
   - wobei die Vorderseite (4) eine maximale Breite (Bmax) aufweist, die parallel zur geraden Oberkante (5) gemessen wird, und eine maximale Höhe (Hmax) aufweist, die senkrecht zur geraden Oberkante (5) gemessen wird,
   - wobei das Verhältnis der Fläche der Vorderseite (4) zu der Fläche eines Rechtecks (9), das durch maximale Breite (Bmax) und maximale Höhe (Hmax) aufgespannt wird, d.h.

$$\frac{Fläche\ (Vorderseite)}{Fläche\ (Rechteck)}$$

, mindestens 0,6 beträgt,

   **dadurch gekennzeichnet, dass** das Verhältnis R von maximaler Breite (Bmax) und maximaler Höhe (Hmax), d.h. Bmax/Hmax, einen Wert zwischen 1,2 und 1,275 aufweist, und wobei das IMD ein implantierbarer Kardioverter-Defibrillator oder ein CRT-Gerät ist.

2. Gerätegehäuse (2) nach Anspruch 1, wobei die maximale Breite (Bmax) einen Wert von 60 mm aufweist.

3. Gerätegehäuse (2) nach einem der vorigen Ansprüche, wobei die maximale Höhe (Hmax) einen Wert 48 mm aufweist.

4. Gerätegehäuse (2) nach einem der vorigen Ansprüche, wobei das Gerätegehäuse (2) eine maximale Dicke (Dmax) aufweist, wobei die Dicke senkrecht zur Ebene der Vorderseite gemessen wird, und die maximale Dicke einen Wert von 9 mm bis 15 mm; oder 9 mm bis 12 mm, oder 10 mm bis 11 mm; oder von 10 mm aufweist.

5. Gerätegehäuse (2) nach einem der vorigen Ansprüche, wobei das Gerätegehäuse (2) eine Rückseite (4') aufweist, die deckungsgleich und gegenüberliegend zur Vorderseite (4) angeordnet ist, und die ebene Stirnseite (6) an eine gerade Oberkante der Rückseite (4') anschließt.

6. Gerätegehäuse (2) nach einem der vorigen Ansprüche, wobei die Außenkontur der Vorderseite (4) Kurven mit mindestens zwei unterschiedlichen Kurvenradien (7, 7', 7'') aufweist, wobei ein maximaler Kurvenradius (8) von 12 cm, oder 11 cm, oder 10 cm, oder 9,5 cm nicht überschritten wird.

7. Gerätegehäuse (2) nach einem der vorigen Ansprüche, wobei das Verhältnis der Fläche der Vorderseite (4) zu der Fläche einer Ellipse (10), die durch maximale Breite (Bmax) und maximale Höhe (Hmax) aufgespannt wird, d.h. $\frac{Fläche\ (Vorderseite)}{Fläche\ (Ellipse)}$, höchstens 1,5; oder höchstens 1,4; oder höchstens 1,3; oder höchstens 1,2; oder höchstens 1,15; oder höchstens 1,1 beträgt.

8. Gerätegehäuse (2) nach einem der vorigen Ansprüche, wobei das Gerätegehäuse (2) zwei Gehäuseschalen umfasst, wobei die erste Gehäuseschale die Vorderseite (4) aufweist und die zweite Gehäuseschale die Rückseite (4') aufweist, wobei die erste und zweite Gehäuseschale symmetrisch ausgebildet sind.

9. Gerätegehäuse (2) nach einem der vorigen Ansprüche, wobei das Gerätegehäuse (2) abgerundete Kanten aufweist.

10. Gehäuseanordnung (1) für ein IMD, umfassend ein Gerätegehäuse (2) nach einem der vorigen Ansprüche und einen Header (3), wobei der Header (3) an der ebenen Stirnseite (6) mit dem Gerätegehäuse (2) verbunden ist.

## Claims

1. A device housing (2) for an IMD, comprising:

   - a front side (4),
   - a flat end face (6) that is arranged perpendicularly to the front side (4) and adjoins a straight upper edge (5) of the front side,
   - the front side (4) having a maximum width (Bmax) that is measured in parallel with the straight upper edge (5) and a maximum height (Hmax) that is measured perpendicularly to the straight upper edge (5),
   - the ratio of the surface area of the front side (4) to the surface area of a rectangle (9) that is spanned by the maximum width (Bmax) and the maximum height (Hmax), $\frac{surface\ area\ (front\ side)}{surface\ area\ (rectangle)}$, being at least 0.6, **characterized in that** the ratio R of maximum width (Bmax) to maximum height (Hmax), i.e. Bmax/Hmax, has a value between 1.2 and 1.275, and the IMD being an implantable cardioverter defibrillator, or a CRT device.

2. The device housing (2) according to claim 1, wherein the maximum width (Bmax) has a value of 60 mm.

3. The device housing (2) according to any one of the preceding claims, wherein the maximum height (Hmax) has a value of 48 mm.

4. The device housing (2) according to any one of the preceding claims, wherein the device housing (2) has a maximum thickness (Dmax), the thickness being measured perpendicularly to the plane of the front side, and the maximum thickness having a value of 9 mm to 15 mm, or 9 mm to 12 mm, or 10 mm to 11 mm, or 10 mm.

5. The device housing (2) according to any one of the preceding claims, wherein the device housing (2) has a rear side (4') that is arranged to be congruent with and opposite the front side (4), and the flat end face (6) adjoins a straight upper edge of the rear side (4').

6. The device housing (2) according to any one of the preceding claims, wherein the outer contour of the front side (4) has curves having at least two different radii of curvature (7, 7', 7"), a maximum radius of curvature (8) of 12 cm, or 11 cm, or 10 cm, or 9.5 cm, not being exceeded.

7. The device housing (2) according to any one of the preceding claims, wherein the ratio of the surface area of the front side (4) to the surface area of an ellipse (10) that is spanned by the maximum width (Bmax) and the maximum height (Hmax), i.e. $\frac{surface\ area\ (front\ side)}{surface\ area\ (ellipse)}$, is at most 1.5, or at most 1.4, or at most 1.3, or at most 1.2, or at most 1.15, or at most 1.1.

8. The device housing (2) according to any one of the preceding claims, wherein the device housing (2) comprises two housing shells, the first housing shell having the front side (4) and the second housing shell having the rear side (4'), the first and second housing shells being configured to be symmetrical.

9. The device housing (2) according to any one of the preceding claims, wherein the device housing (2) has rounded edges.

10. A housing arrangement (1) for an IMD, comprising a device housing (2) according to any one of the preceding claims and a header (3), wherein the header (3) is connected to the device housing (2) at the flat end face (6).

## Revendications

1. Boitier d'appareil (2) pour un dispositif médical implantable IMD, présentant :

   - une face avant (4),
   - une face frontale (6) plane qui est disposée perpendiculairement par rapport à la face avant (4) et se raccorde à un bord supérieur (5) rectiligne de la face avant,

- dans lequel la face avant (4) présente une largeur maximale (Bmax) qui se mesure parallèlement par rapport au bord supérieur (5) rectiligne et une hauteur maximale (Hmax) qui se mesure perpendiculairement par rapport au bord supérieur (5) rectiligne,

- dans lequel le ratio de la surface de la face avant (4) sur la surface d'un rectangle (9) qui est défini par la largeur maximale (Bmax) et la hauteur maximale (Hmax), c'est-à-dire $\frac{surface\ (face\ avant)}{surface\ (rectangle)}$ est égal à au moins 0,6, **caractérisé en ce que** le rapport R de la largeur maximale (Bmax) sur la hauteur maximale (Hmax), c'est-à-dire $\frac{Bmax}{Max}$, présente une valeur entre 1,2 et 1,275, et dans lequel l'IMD est un défibrillateur-cardioverteur implantable ou un appareil de resynchronisation cardiaque CRT.

2. Boitier d'appareil (2) selon la revendication 1, dans lequel la largeur maximale (Bmax) présente une valeur de 60 mm.

3. Boitier d'appareil (2) selon l'une des revendications précédentes, dans lequel la hauteur maximale (Hmax) présente une valeur de 48 mm.

4. Boitier d'appareil (2) selon l'une des revendications précédentes, le boitier d'appareil (2) présentent une épaisseur maximale (Dmax), dans lequel l'épaisseur est mesurée perpendiculairement par rapport au plan de la face avant, et l'épaisseur maximale présente une valeur de 9 mm à 15 mm ; ou de 9 mm à 12 mm, ou de 10 mm à 11 mm ; ou de 10 mm.

5. Boitier d'appareil (2) selon l'une des revendications précédentes, le boitier d'appareil (2) présentant une face arrière (4') qui est disposée superposée et en face de la face avant (4), et la face frontale (6) plane est adjacente à un bord supérieur rectiligne de la face arrière (4').

6. Boitier d'appareil (2) selon l'une des revendications précédentes, dans lequel le contour extérieur de la face avant (4) présente des courbes avec au moins deux rayons de courbure (7, 7', 7") différents, où un rayon de courbure maximal (8) de 12 cm, ou de 11 cm, ou de 10 cm, ou de 9,5 cm n'est pas dépassé.

7. Boitier d'appareil (2) selon l'une des revendications précédentes, dans lequel le ratio de la surface de la face avant (4) sur la surface d'une ellipse (10) qui est définie par la largeur maximale (Bmax) et la hauteur maximale (Hmax), c'est-à-dire $\frac{surface\ (face\ avant)}{surface\ (ellipse)}$, est égal à au plus à 1,5 ; ou au plus à 1,4 ; ou au plus à 1,3 ; ou au plus à 1,2 : ou au plus à 1,15 ; ou au plus à 1,1.

8. Boitier d'appareil (2) selon l'une des revendications précédentes, le boitier d'appareil (2) comprenant deux coques de boitier, où la première coque de boitier présente la face avant (4) et la deuxième coque de boitier présente la face arrière (4'), où les première et deuxième coques de boitier sont conçues symétriques.

9. Boitier d'appareil (2) selon l'une des revendications précédentes, le boitier d'appareil (2) présentant des bords arrondis.

10. Ensemble boitier (1) pour un IMD comprenant un boitier d'appareil (2) selon l'une des revendications précédentes et un header (3), où le header (3) est relié à la face frontale (6) plane avec le boitier d'appareil (2).

EP 3 705 158 B1

**FIG. 1**

**FIG. 2**

EP 3 705 158 B1

Bmax

8

7'

Hmax

**FIG. 3**

FIG. 4

FIG. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070060980 A1